# EUROPÄISCHE PATENTSCHRIFT

(11) **EP 1 038 949 B1**
(45) Veröffentlichungstag und Bekanntmachung des Hinweises auf die Patenterteilung: **02.05.2003**
(21) Anmeldenummer: 00810172.7
(22) Anmeldetag: 01.03.2000
(51) Int. Cl.: C12M 1/04, B65B 31/04

(54) **Gerät zur Erzeugung einer anaeroben, mikroaerophilen oder einer anderen Atmosphäre in einem geschlossenen Wechselbehälter**
Installation for the production of an anaerobic, microaerophilic or other atmosphere in a closed interchangeable recipient
Installation pour la production d'une atmosphère anaérobique, microaérophilique ou autre dans un récipient fermé et interchangeable

(30) Priorität: 16.03.1999 CH 50399
(43) Veröffentlichungstag der Anmeldung: 27.09.2000
(73) Patentinhaber: Jenny, Alois, 6215 Beromünster (CH)
(72) Erfinder: Jenny, Alois, 6215 Beromünster (CH)
(74) Vertreter: Luchs, Willi

(56) Entgegenhaltungen:
- EP-A- 0 460 979
- US-A- 4 188 265
- US-A- 5 454 421
- US-A- 5 588 970

## Beschreibung

Die Erfindung betrifft ein Gerät nach dem Oberbegriff des Anspruchs 1.

Derartige Atmosphären werden bekanntlich in der Mikrobiologie, der Medizin der Pharmazie und in der Forschung für die Anzüchtung von anaeroben, mikroaerophilen und anderen Mikroorganismen benötigt. Hierfür wird der Wechselbehälter mit den Laborproben (auf Nährböden in Petrischalen) bestückt und anschliessend in die vorgesehene Öffnung des Gerätes gestellt, welches den Wechselbehälter automatisch ankoppelt und durch programmgesteuertes Evakuieren und Begasen die gewünschte Atmosphäre erzeugt. Nach Programmablauf wird der Wechselbehälter dem Gerät entnommen und in den Wärmeschrank zur Bebrütung gestellt.

Bei einem aus dem Prospekt "MART Systeme - weltweit ein Standard in der Mikrobiologie" beschriebenen "Anoxomat" wird eine Steuervorrichtung zur Teilautomatisierung der von Begasung und Evakuierung der eingangs genannten Art beschrieben. Diese Steuervorrichtung ist mit freien Anschlussschläuchen versehen, an denen ein Gefäss zur Erzeugung der anaeroben Atmosphäre anschliessbar ist. Die entsprechenden Anschlussorgane sind auf dem Gefässdeckel als manuelle Kupplungsteile vorhanden. Dazu sind mehrere Kabel, Anschlussschläuche und Anschlussorgane für die Steuerung, Vakuumpumpe und Gasflaschen vorgesehen. Dies verursacht eine umständliche Handhabung und zudem muss die Vakuumpumpe, da sie nicht in der Steuervorrichtung integriert ist, irgendwo extern aufgestellt werden. Es besteht die Gefahr einer fehlerhaften Funktion infolge einer Verwechslung der Anschlussschläuche bzw. der ihnen zugeordneten Anschlüsse. Weiter sind die "fliegenden" Anschlussschläuche und Kabel resp. die manuellen Anschlussorgane der Gefässdeckels und der Steuervorrichtung sowie der Vakuumpumpe exponiert und dadurch sehr beschädigungsanfällig.

Bei einer bekannten Einrichtung gemäss der US-A-5,424,421 wird in einer Gasflasche ein Vakuum erzeugt und anschliessend dieselbe gefüllt. Diese Einrichtung enthält in ihrem Zentrum einen um seine Achse drehbaren Schaft, durch welchen ein die Flasche öffnendes Ventil durch ein Anheben betätigbar ist. Der Schaft ist hierbei durch einen pneumatisch betätigbaren Kolben drehbar gehalten. Mit dieser Einrichtung ist insbesondere in der Herstellung eine relativ komplizierte Konstruktion geschaffen worden, bei welcher ein solches Betätigungsglied erforderlich ist, um das Ventil der Flasche zu öffnen. Ausserdem ist eine dazu separate Gaszuführung in dieser Einrichtung vorzusehen. Zudem muss diese Einrichtung durch ein Befestigungsmittel an den Verschluss der Flasche angeschlossen werden, was mit einer Handmanipulation verbunden ist. Eine solche Gasflasche eignet sich nicht als Wechselbehälter, da sie ohnehin als geschlossenes Gefäss ausgebildet ist.

Bei einem Verfahren zur Erzeugung eines Vakuums in einer Batterie gemäss der Druckschrift US-A-5,588,970 ist eine Zylinder-Kolbeneinheit vorgesehen, von welcher der Kolben bei der oberen Öffnung der Batterie durch eine Feder aufdrückbar ist. Mittels eines Vakuumanschlusses kann die Luft aus der Batterie abgesogen werden. Sobald ein gewisser Unterdruck erzeugt ist, wird die Öffnung im Kolben durch ein Absenken des Zylinders und damit eines Schliessventils geschlossen und ein Elektrolyt in die Batterie gefüllt. Anschliessend wird die obere Öffnung der Batterie irreversibel geschlossen. Es ist damit nicht ein Wechselbehälter vorgesehen, in den entsprechende Proben hineingestellt werden können und der derart ausgebildet ist, dass er wiederverwendbar und mit entsprechenden Anschlüssen versehen ist, die es ermöglichen, dass er laufend wieder mit einer neuen Gasatmosphäre beschickt werden kann.

Der Erfindung liegt die Aufgabe zugrunde, diese Nachteile zu beheben und ein Gerät der eingangs genannten Art zu schaffen, das sich dadurch auszeichnet, dass bei ihm ein mobiler Wechselbehälter mit den Laborproben einfach, schnell und prozesssicher in die Öffnung des stationären Gerätes gestellt werden kann und anschliessend die gewünschte Atmosphäre im Innenraum des Wechselbehälters ohne nennenswerten manuellen Aufwand erzeugt wird.

Diese Aufgabe wird erfindungsgemäss durch den kennzeichnenden Teil des Anspruchs 1 gelöst.

Bei einer sehr vorteilhaften Ausführung ist das stationäre Gerät mit einer integrierten Vakuumpumpe, verschiedenen Gasanschlüssen, Gasventilen sowie einem vakuumbetriebenen Andockzylinder versehen, dabei wird der Wechselbehälter durch den Andockzylinder gasdicht angekoppelt, anschliessend wird der Wechselbehälter gemäss einem Programm ein- oder mehrfach evakuiert und begast, bis die gewünschte Atmosphäre im Innenraum des Wechselbehälters erzeugt ist.

Beim erfindungsgemässen Gerät ist es lediglich erforderlich, den die Laborproben enthaltenden Wechselbehälter in die vorgesehene Öffnung zu bringen und dann den Startknopf zu betätigen. Danach erfolgen Ankopplung, Evakuierung und Begasung zur Erzeugung der gewünschten Atmosphäre ohne weiteres manuelles Zutun vollautomatisch. Die Prozesssicherheit des Gerätes wird auch dadurch gewährleistet, dass weder Schläuche, noch manuelle Kupplungselemente vorhanden sind und dass beim Andockzylinder die Gaszuführung über einen internen Kanal erfolgt. Am Wechselbehälter selbst sind ebenfalls keine vorstehenden Anschlussorgane vorhanden. Daraus resultiert weiter ein Vorteil, dass die Wechselbehälter gestapelt, und somit sehr platzsparend im Wärmeschrank angeordnet werden können. Der vakuumbetriebene Andockzylinder ermöglicht zudem eine einfache und raumsparende Bauweise des stationären Gerätes.

Es ist von Vorteil, wenn der Andockzylinder erfindungsgemäss durch eine Zylinder-Kolbeneinheit gebildet ist, wobei der Zylinder im Gerät befestigt ist und der Kolben wechselbehälterseitig einen aus dem Zylinder herausragenden Andockvorsatz aufweist, der gegen eine koaxiale Druckfeder verschiebbar ist und stirnseitig mit einer gegen den Wechselbehälterdeckel andrückbaren Dichtring versehen ist. Kolben und Andockvorsatz sind mit einem intern durchgehenden Kanal für Evakuierung und Gaszuführung ausgestattet. Durch diese Konstruktion ist der Andockzylinder weitgehend wartungsfrei und prozesssicher aufgebaut.

Um das Greifen und Handeln des Wechselbehälters zu erleichtern, sieht die Erfindung vor, dass der Wechselbehälter aus leichtem Material (Polycarbonat, Alu) und mit umfänglich verteilt angeordneten Vertikalrippen versehen ist.

Zwecks eines möglichst einfachen, wartungsfreien Verschliessens des Wechselbehälters ist es ferner vorgesehen, dass der Wechselbehälterdeckel mit einem Bajonettverschluss ausgerüstet ist, welcher zweckmässigerweise zum Innenrand des Wechselbehälters mit einer Lippendichtung versehen ist und mittig des Verschlusses, in der Position des Andockzylinders angeordnete Gaseinund -auslasskanäle aufweist, in welchen je ein Rückschlagventil eingebaut ist.

Die Erfindung sieht auch vor, dass der Wechselbehälterboden mit einer mittigen Absenkung versehen ist, welche mit einer entsprechend ausgebildeten Erhebung des Wechselbehälterdeckels korrespondiert. Auf diese Weise sind die Wechselbehälter zentriert und einfach zueinander stapelbar.

Weitere Merkmale der Erfindung ergeben sich aus den Patentansprüchen und der nachfolgenden Beschreibung.

Im folgenden werden zwei Ausführungsformen der Erfindung unter Bezugnahme auf die Zeichnung näher beschrieben. Es zeigen:
- Fig. 1: ein erfindungsgemässes Gerät zur Erzeugung anaerober, mikroaerophiler oder anderen Atmosphären in einem geschlossenen Wechselbehälter, in der Vorderansicht dargestellt,
- Fig. 2: das Gerät nach Fig. 1, in einem Querschnitt entlang der Linie II-II in Fig. 1 dargestellt,
- Fig. 3: den Teilquerschnitt III aus Fig. 2, vergrössert dargestellt,
- Fig. 4: den Andockzylinder des Geräts nach den Figuren 1 und 2, in der Draufsicht dargestellt,
- Fig. 5: der Pneumatikplan für das Gerät nach den Figuren 1 bis 4, schematisch dargestellt,
- Fig. 6: einen Teilschnitt entlang der Linie VI - VI in Fig.3, und
- Fig. 7: eine zweite Ausführungsform des Geräts als "Dreieckprofil" mit Anordnung der internen Aggregate in den beiden Seitenteilen des Gehäuses.

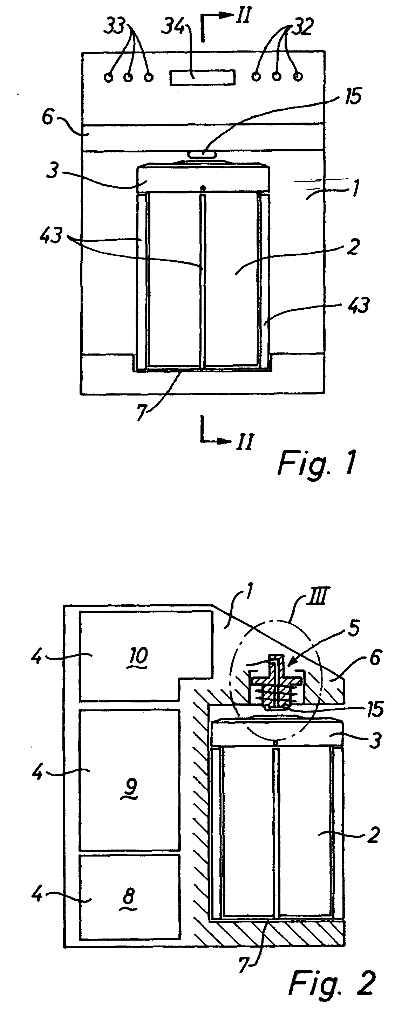

Die Figuren 1 bis 5 zeigen ein stationäres Gerät 1 zur Erzeugung anaerober, mikroaerophiler oder anderen Atmosphären in einem geschlossenen Wechselbehälter 2, der mit einem Deckel 3 versehen ist. Das Gerät 1 ist mit einer Einrichtung 4 zur Erzeugung der gewünschten Atmosphäre im Innenraum des Wechselbehälters ausgestattet. Der Wechselbehälter 2 ist deckelseitig an die Einrichtung 4 mittels eines vakuumbetriebenen Andockzylinders 5 koppelbar, der in einer Aufnahme 6 des Geräts 1 eingebaut ist. Unterhalb der Aufnahme 6 ist im Gerät 1 eine Öffnung 7 zur Positionierung des Wechselbehälters angeordnet.

Die Einrichtung 4 setzt sich aus drei Baueinheiten 8, 9 und 10 zusammen, wobei die Baueinheit 8 mit einer Vakuumpumpe 24 versehen ist, indessen die Baueinheit 9 mit mindestens einer Gasversorgungsquelle 26a, 26b, mit dem Andockzylinder 5, Schaltventilen 29a bis 29e und einem Vakuumsensor 31 mit den dazugehörigen Sensoren ausgerüstet ist. Der Steuer- und Programmiereinheit 10 sind Drucktasten 32, Anzeige 33 und ein Drucker 34 zugeordnet.

Erfindungsgemäss weist das Gerät 1 Mittel für ein automatisiertes Verbinden bzw. Lösen der Einrichtung 4 zum bzw. vom Innenraum des Wechselbehälters 2 auf, wobei durch diese Mittel ermöglicht wird, dass die Wechselbehälter 2 nacheinander in der Art eines Wechselsystems durch jeweiliges Evakuieren und Begasen mit der gewünschten Atmosphäre im Wechselbehälterinnenraum versehen werden.

Als Mittel für dieses automatisierte Verbinden bzw. Lösen der Einrichtung 4 zum bzw. vom Innenraum des Wechselbehälters 2 ist ein vakuumbetriebener Andockzylinder 5 vorgesehen, der durch eine Zylinder-Kolbeneinheit 11, 12 gebildet ist, wobei der Zylinder 11 in der Aufnahme 6 des Geräts 1 ortsfest befestigt ist und der Kolben 12 wechselbehälterseitig einen aus dem Zylinder 11 herausragenden Andockvorsatz 13 aufweist, der zusammen mit dem Kolben 12 gegen eine koaxiale Druckfeder 14 verschiebbar ist und stirnseitig mit einer gegen den Wechselbehälterdeckel 3 andrückbaren Dichtlippe 15 versehen ist. Der Andockvorsatz 13 ist gasdicht in einer behälterseitigen Stirnwand 16 des Zylinders 11 geführt, und die Druckfeder 14 ist im Zylinder zwischen dem Kolben 12 und der Stirnwand 16 eingelegt.

Der Kolben 12 ist auf der vom Wechselbehälter 2 abgekehrten Kolbenseite mit einem den Gasleitungsanschluss 17 des Andockzylinders 5 aufweisenden Kolbenzapfen 18 versehen, der aus dem dort stirnseitig offenen Zylinder 11 herausragt. Der Kolben 12, der Andockvorsatz 13 und der Kolbenzapfen 18 sind koaxial zueinander angeordnet und von einem axial verlaufenden Gasein- und - auslasskanal 19 durchdrungen, der in den Gasleitungsanschluss 17 der Schnellkupplung 5 mündet. Der Zylinder 11 ist nahe seiner behälterseitigen Stirnwand 16 mit einem Vakuumleitungsanschluss 20 versehen. Die Dichtung 15 des Andockvorsatzes 13 ist durch eine O-Ringdichtung gebildet, die in einer Ringnut 21 des Andockvorsatzes 13 gehalten ist und diesen in Hubrichtung des Kolbens 12 stirnseitig überragt.

Der Zylinder 11 ist in der Aufnahme 6 mit Schrauben befestigt, die in Gewindebohrungen 22 des Zylinders 11 einschraubbar sind. Am stirnseitig offenen Zylinderende sind zwei Anschläge 23 zur Begrenzung des Kolbenhubes vorgesehen.

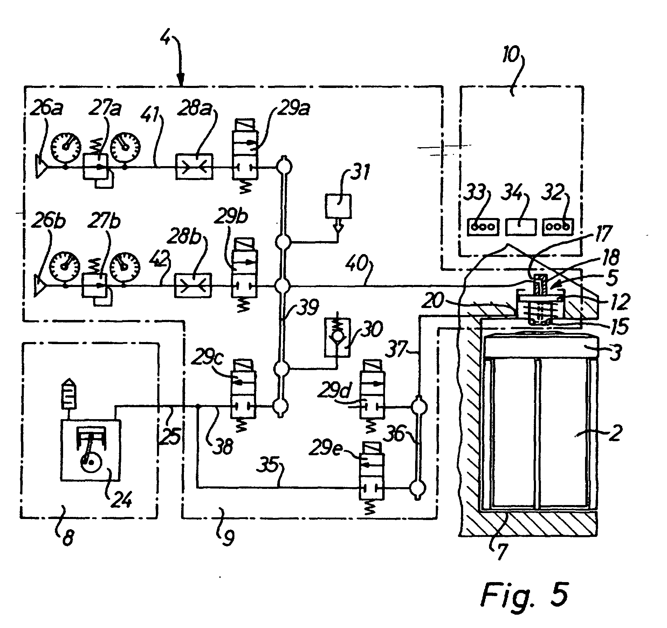

Gemäss Figur 5 ist die Vakuumpumpe 24 der Baueinheit 8 über eine Leitung 25 an die Baueinheit 9 angeschlossen. Letztere ist mit den Gasversorgungsquellen 26a und 26b, den Druckreglern 27a und 27b, den Drosseln 28a und 28b, den als Wegeventile ausgebildeten Schaltventilen 29a bis 29e, sowie dem Überdruckventil 30 und dem Vakuumsensor 31 ausgestattet. Diese Elemente stehen mit der Steuer- und Programmiereinheit 10 mit den Drucktasten 32, den Anzeigen 33 und dem Drucker 34 in Wirkverbindung.

Die Vakuumpumpe 24 ist einerseits über die Leitungen 25 und 35, das Schaltventil 29e, einen Verteiler 36 und eine Leitung 37 mit dem Vakuumleitungsanschluss 20 des Zylinders 11 und andererseits über die Leitungen 25 und 38, das Schaltventil 29c, einen Verteiler 39 und eine Leitung 40 mit dem Gasleitungsanschluss 17 des Kolbenzapfens 18 verbindbar. Der Vakuumleitungsanschluss 20 ist auch über die Leitung 37, den Verteiler 36 und das Schaltventil 29d mit der Aussenatmosphäre verbindbar.

Die Druckversorgungsquellen 26a und 26b liefern das zur Erzeugung der anaeroben Atmosphäre jeweils gewünschte Gas oder Gasgemisch, dessen genaue Dosierung mit Hilfe der Druckregler 27a und 27 b sowie der Drosseln 28a und 28b genau einstellbar ist.

In dem beschriebenen Ausführungsbeispiel liefert die Quelle 26a reines N2 und die Quelle 26b ein Gasgemisch aus N2, H2 und CO2. Die Einrichtung 4 kann selbstverständlich auch mit einer einzigen oder aber mit mehr als zwei Gasversorgungsquellen versehen sein. Es könnte auch ein Gasgemisch annähernd nur aus CO2 vorgesehen sein.

Die Druckversorgungsquelle 26a ist über eine Leitung 41, das Schaltventil 29a, den Verteiler 39 und die Leitung 40 mit dem Gasleitungsanschluss 17 des Kolbenzapfens 18 verbindbar. In ähnlicher Weise kann auch die Druckversorgungsquelle 26b über eine Leitung 42, das Schaltventil 29b, den Verteiler 39 und die Leitung 40 mit dem Gasleitungsanschluss 17 des Kolbenzapfens 18 verbunden werden. Das Überdruckventil 30 und der Vakuumsensor 31 sind beide an den Verteiler 39 angeschlossen.

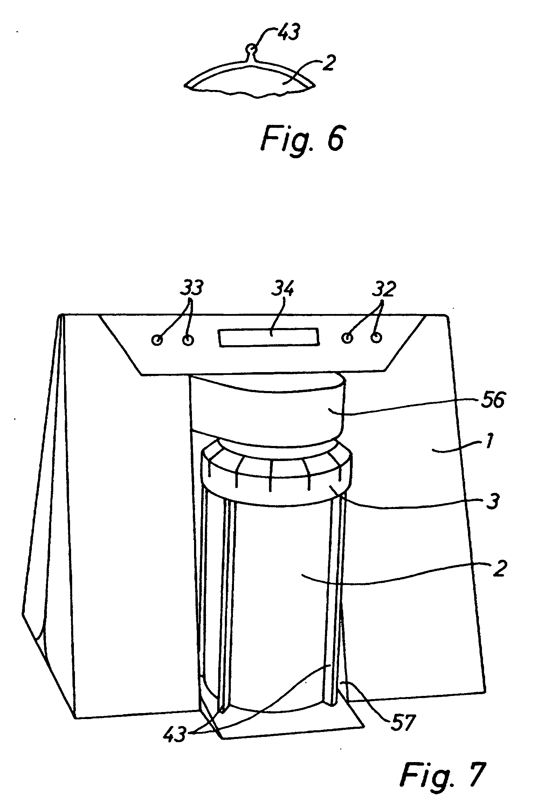

Der Wechselbehälter 2 für das Gerät gemäss der vorstehenden Beschreibung ist topfförmig ausgebildet und weist umfänglich angeordnete Vertikalrippen 43 auf, mit Hilfe derer man der Wechselbehälter 2 leicht handhaben und in die Öffnung 7 stellen kann. Wie aus Figur 6 ersichtlich, sind die Vertikalrippen 43 an ihren Aussenrändern abgerundet. Der Wechselbehälterdeckel 3 ist als ein gasdicht dem Innenumfang des Wechselbehälters 2 angepasster Scheibenteller 44 ausgebildet, der mit einem den Wechselbehälterrand 45 umgreifenden Befestigungsring 46 mit Bajonettverschluss 47 versehen ist. Die Vertikalrippen 43 des Wechselbehälters 2 sind in ihrer Breite so bemessen, dass sie umfänglich mit dem Befestigungsring 46 bündig sind. Der Scheibenteller 44 ist umfänglich mit einer Lippendichtung 48 versehen und weist mittig angeordnete Gasein- und -auslasskanäle 49, 50 auf, in welchen je ein Rückschlagventil 51, 52 eingebaut ist. Der Wechselbehälterboden ist mit einer mittigen Absenkung 53 versehen, welche mit einer entsprechend ausgebildeten Erhebung 54 des Wechselbehälterdeckels 3 korrespondiert. Die ineinanderpassenden Absenkungen 53 und Erhebungen 54 erleichtern das Übereinanderstapeln der Wechselbehälter. Der Scheibenteller 44 weist auch einen Katalysator 55 auf, der in den Innenraum des Wechselbehälters hineinragt.

Das Gerät nach Figur 7 unterscheidet sich vom Gerät nach den Figuren 1 bis 6 im wesentlichen nur dadurch, dass das Seitenprofil ein Dreieck darstellt und sämtliche internen Aggregate in den beiden Seitenteilen untergebracht und wieder mit einer Öffnung 57 für die Aufnahme des Wechselbehälters 2 versehen sind.

Das vorstehend beschriebene Gerät arbeitet wie folgt:

Zunächst wird der Wechselbehälter 2 mit den zu behandelnden Laborproben bestückt (Petrischalen), mit dem Deckel 3 gasdicht verschlossen und in die Öffnung 7 bzw. 57 unterhalb der Aufnahme 6 bzw. 56 des Andockzylinders positioniert.

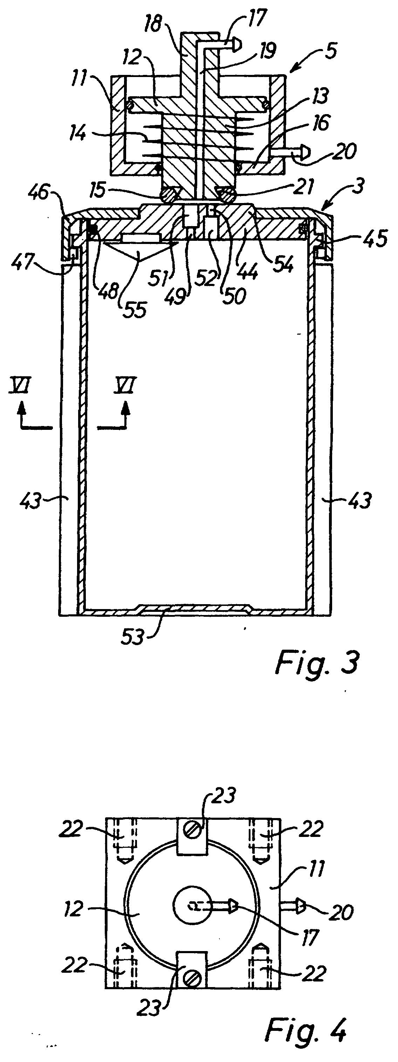

Danach wird durch eine Drucktaste 32 das Programm gestartet, welches vollautomatisch durchläuft. Dabei wird zuerst der Andockzylinder 5 aktiviert, indem der Zylinder 11 durch das Schaltventils 29e mit der Vakuumpumpe 24 verbunden wird. Dadurch entsteht im Zylinder 11 ein Vakuum, das eine Verschiebung des Kolbens 12 samt Andockvorsatz 13 verursacht, bis die Dichtlippe 15 - wie in Fig. 3 ersichtlich - gasdicht gegen den mittleren Bereich des Wechselbehälterdeckels 3 mit den Gasein- und -auslasskanälen 49, 50 angedrückt ist und durch ein Schliessen des Schaltventils 29e angedrückt bleibt. Hierbei entsteht zwischen den gegenüberliegenden Stirnwänden des Andockvorsatzes 13 und des Wechselbehälterdeckels 3 eine zur Aussenatmosphäre hin gasdicht abgeschlossene Gaskammer, die einerseits mit dem Gasleitungsanschluss 17 des Kolbens 12 und andererseits über die Gasein- und -auslasskanäle 49, 50 mit dem Innenraum des Wechselbehälters 2 verbunden ist. Auf diese Weise ist der Wechselbehälter 2 sowohl an die Vakuumpumpe 24 als auch an eine der Gasversorgungsquellen 26a, 26b der Einrichtung 4 angeschlossen.

Zur Erzeugung der anaeroben Atmosphäre wird zunächst die Verbindung zur Vakuumpumpe 24 durch das Schaltventil 29c hergestellt. Hierbei wird der Innenraum des Wechselbehälters 2 über den Gasauslasskanal 50, den Gasleitungskanal 19 und den Gasleitungsanschluss 17 evakuiert. Nach Erreichen des gewünschten Vakuums, gemessen durch den Vakuumsensor 31 im Wechselbehälter wird nunmehr die Verbindung zu der Vakuumpumpe 24 mit dem Schaltventil 29c wieder unterbrochen und eine Verbindung zur einer der Gasversorgungsquellen 26a, 26b durch Schalten der Ventile 29a oder 29b hergestellt. Auf diese Weise wird das jeweils vorgesehene Gas oder Gasgemisch dem Innenraum des Wechselbehälters 2 zugeführt, bis dort die gewünschte Atmosphäre aufgebaut ist. Während der ganzen Operation ist der gasdichte Anschluss des Wechselbehälters 2 an die Vakuumpumpe 24 bzw. an die Gasversorgungsquellen 26a, 26b durch den vakuumbetriebenen Andockzylinder 5 sichergestellt.

Zur Kontrolle und Anzeige des Vakuums bzw. des aktuellen Prozesszustandes dienen der Vakuumsensor 31 und die Anzeigen 33. Nachdem im Wechselbehälterinnerraum die gewünschte Atmosphäre vorhanden ist, wird nunmehr der Andockzylinder 5 wieder gelöst. Hierfür wird das Schaltventil 29d geschaltet. Somit ist eine Belüftungsverbindung zwischen dem Vakuumzylinder 11 zur Aussenatmosphäre hergestellt. Die Druckfeder 14 fährt den Kolben 12 und den Andockvorsatz 13 mit der Dichtlippe 15 wieder zurück, und der Andockzylinder 5 ist somit wieder gelöst. Hernach kann man den Wechselbehälter 2 aus dem Gerät 1 herausnehmen und z.B. in einen Wärmeschrank zur Bebrütung der Proben stellen. Sodann kann bereits wieder ein nächster Wechselbehälter angedockt und mit der gewünschten Atmosphäre versehen werden.

Selbstverständlich könnten die erfindungsgemässen Mittel für ein automatisiertes Verbinden bzw. Lösen der Einrichtung 4 zum bzw. vom Innenraum des Wechselbehälters 2 anders als durch die oben erläuterte Zylinder-Kolbeneinheit realisiert sein, wie zum Beispiel durch eine elektromechanische Lösung oder dergleichen.

## Patentansprüche

1. Gerät zur Erzeugung einer anaeroben, mikroaerophilen oder einer anderen Atmosphäre in einem geschlossenen Wechselbehälter, mit einem einen Deckel (3) aufweisenden Wechselbehälter (2), der an eine Einrichtung (4) zur Erzeugung der gewünschten Atmosphäre im Innenraum des Wechselbehälters anschliessbar ist, zwecks Bebrütung von in den Wechselbehälter (2) gelegten Mikroorganismen, **dadurch gekennzeichnet, dass**
das Gerät (1) Mittel für ein automatisiertes Verbinden bzw. Lösen der Einrichtung (4) zum bzw. vom Innenraum des Wechselbehälters (2) aufweist, wobei durch diese Mittel ermöglicht wird, dass die Wechselbehälter (2) nacheinander in der Art eines Wechselsystems durch jeweiliges Evakuieren und Begasen mit der gewünschten Atmosphäre im Wechselbehälterinnenraum versehen werden, *wobei der wegnehmbare Deckel (3) des Wechselbehälters (2) Gaseinlass-und Gasauslass kanäle (49, 50) mit je einem eingebauten Rückschlagventil (51, 52) oder dergleichen für den Gasaustausch enthält*.

2. Gerät nach Anspruch 1, **dadurch gekennzeichnet, dass** als Mittel für das automatisierte Verbinden bzw. Lösen der Einrichtung (4) zum bzw. vom Innenraum des Wechselbehälters (2) ein vakuumbetriebener Andockzylinder (5) vorgesehen ist, der durch eine Zylinder-Kolbeneinheit (11, 12) gebildet ist, einen internen Gasführungskanal (19) mit Gasleitungsanschluss (17) für die Einrichtung (4) zur Erzeugung der gewünschten Atmosphäre aufweist und gasdicht gegen einen Gasein- und -auslässe (49, 50) aufweisenden Bereich (54) des Wechselbehälterdeckels (3) andrückbar ist.

3. Gerät nach Anspruch 2, **dadurch gekennzeichnet, dass** der Zylinder (11) im Gerät ortsfest befestigt ist und der Kolben (12) behälterseitig einen aus dem Zylinder (11) herausragenden Andockvorsatz (13) aufweist, der zusammen mit dem Kolben (12) gegen eine koaxiale Druckfeder (14) veschiebbar ist und stirnseitig mit einer gegen den Wechselbehälterdeckel (3) andrückbaren Dichtlippe (15) versehen ist.

4. Gerät nach Anspruch 3, **dadurch gekennzeichnet, dass** der Andockvorsatz (13) gasdicht in einer behälterseitigen Stirnwand (16) des Zylinders (11) geführt ist und die Druckfeder (14) im Zylinder (11) zwischen dem Kolben (12) und der Stirnwand (16) des Zylinders (11) eingelegt ist.

5. Gerät nach Anspruch 3 oder 4, daduch gekennzeichnet, dass der Kolben (12) auf der vom Wechselbehälter (2) abgekehrten Kolbenseite mit einem den Gasleitungsanschluss (17) des Andockzylinders (5) aufweisenden Kolbenzapfen (18) versehen ist.

6. Gerät nach Anspruch 5, **dadurch gekennzeichnet, dass** der Kolben (12), der Andockvorsatz (13) und der Kolbenzapfen (18) koaxial zueinander angeordnet sind und von einem axial verlaufenden Gasein- und -auslasskanal (19) durchdrungen sind, der in den Gasleitungsanschluss (17) des Andockzylinders (5) mündet.

7. Gerät nach einem oder mehreren der Ansprüche 1 bis 6, **dadurch gekennzeichnet, dass** im Gerät (1) eine erste Baueinheit (8) mit einer Vakuumpumpe (24), eine zweite Baueinheit (9) mit mindestens einer Gasversorgungsquelle (26a, 26b), dem Andockzylinder (5), Schaltventilen (29a bis 29e) und Regler (31), sowie eine dritte Baueinheit (10) mit Drucktasten (32), Anzeigen (33) und Drucker (34) eingebaut sind.

8. Wechselbehälter für ein Gerät nach einem oder mehreren der Ansprüche 1 bis 7, **dadurch gekennzeichnet, dass** der *wegnehmbare* Wechselbehälterdeckel (3) *Gaseinlass*- *und Gasauslasskanäle (49, 50)* mit je einem eingebauten Rückschlagventil (51,52) oder dergleichen für den Gasaustausch via Andockzylinder (5) enthält.

9. Wechselbehälter nach Anspruch 8, **dadurch gekennzeichnet, dass** der Wechselbehälter (2) topfförmig ist und mit umfänglich verteilt angeordneten Vertikalrippen (43) versehen ist.

10. Wechselbehälter nach Anspruch 9, **dadurch gekennzeichnet, dass** der Wechselbehälterdeckel durch einen gasdicht dem Innenumfang des Wechselbehälters angepassten Scheibenteller (44) mit einem den Wechselbehälterrand (45) umgreifenden Befestigungsring (46) mit Bajonettverschluss (47) oder dergleichen gebildet ist.

## Claims

1. Device for the production of an anaerobic, micro-aerophilic or another atmosphere in a closed interchangeable container, with an interchangeable container (2) exhibiting a lid (3), which can be connected to a unit (4) for the production of the desired atmosphere in the interior of the interchangeable container for the purpose of the incubation of micro-organisms placed in the interchangeable container (2) **characterised in that**
the device (1) exhibits elements for automatic connecting and, respectively, detaching of the unit (4) with and, respectively, from the interior of the interchangeable container (2), whereby these elements make it possible for the interchangeable containers (2) to be provided together in a form of alternating system for the creation of the desired atmosphere in the interior of the interchangeable container by evacuation and gassing, whereby the detachable lid (3) of the interchangeable container (2) having gas inlet and outlet channels (49, 50) with each having fitted a back-pressure valve (51, 52) or the like for the gas exchange.

2. Device in accordance with claim 1, **characterised in that** a vacuum-operated docking cylinder (5) is provided as the element for automatic connecting and, respectively, detachment of the unit (4) with and, retrospectively, from the interior of the interchangeable container (2) that takes the form of a cylinder-piston unit (11,12), exhibits an internal gas supply channel (19) with gas pipe connection (17) for the unit (4) for the production of the desired atmosphere and is pressed gas-tight against an area (54) of the interchangeable container lid (3) exhibiting gas inlets and outlets (49, 50).

3. Device in accordance with claim 2, **characterised in that** the cylinder (11) is fixed at a specific position in the device and the piston (12) exhibits, on the container side, a docking attachment (13) protruding from the cylinder (11), which, together with the piston (12), can be moved against a coaxial pressure spring (14) and is provided, at the front, with a sealing lip (15) that can be pressed against the lid of the interchangeable container (3).

4. Device in accordance with claim 3, **characterised in that** the docking attachment (13) is supported gas-tight in a front wall (16) of the cylinder (11) at the container side and the pressure spring (14) is inserted in the cylinder (11) between the piston (12) and the front wall (16) of the cylinder (11).

5. Device in accordance with claim 3 or 4, **characterised in that** the piston (12) is provided on the piston side turned away from the interchangeable container (2) with a piston pin (18) exhibiting the gas pipe connection (17) of the docking cylinder (5).

6. Device in accordance with claim 5, **characterised in that** the piston (12), the docking attachment (13) and the piston pin (18) have a coaxial arrangement with one another and are penetrated by an axial gas inlet and outlet channel (19) that opens up into the gas pipe connection (17) of the docking cylinder.

7. Device in accordance with one or several of the claims 1 to 6, **characterised in that**, in the device (1), a first structural element (8) with a vacuum pump (24), a second structural element (9) with at least one gas supply source (26a, 26b), the docking cylinder (5), switching valves (29a to 29e) and regulator (31) as well as a third structural element (10) with push buttons (32), displays (33) and printer (34) are installed.

8. Interchangeable container for a device in accordance with one or several of claims 1 to 7, **characterised in that** the detachable interchangeable container lid (3) having gas inlet and outlet channels (49, 50) with each having fitted a back-pressure valve (51, 52) or the like for the gas exchange via the docking cylinder (5).

9. Interchangeable container in accordance with claim 8, **characterised in that** the interchangeable container (2) takes the form of a pot and is provided with vertical ribs (43) arranged over its circumference.

10. Interchangeable container in accordance with claim 9, **characterised in that** the lid of the interchangeable container takes the form of a disk divider (44) tailored, gas-tight, to the inner circumference of the interchangeable container with a fastening ring (46) embracing the edge of the interchangeable container (45) with bayonet closure (47) or such like.

## Revendications

1. Appareil pour la production d'une atmosphère anaérobique, microaérophilique ou d'une autre atmosphère dans un récipient interchangeable fermé, comprenant un récipient interchangeable (2) présentant un couvercle (3), ledit récipient pouvant être raccordé à un dispositif (4) pour produire l'atmosphère voulue dans l'espace intérieur du récipient interchangeable en vue de la culture de micro-organismes placés dans le récipient interchangeable (2), **caractérisé en ce que** l'appareil (1) présente des moyens pour une liaison et une séparation automatisées du dispositif (4), respectivement avec et vis-à-vis de l'espace intérieur du récipient interchangeable (2), lesdits moyens permettant que les récipients interchangeables (2) soient pourvus les uns après les autres de l'atmosphère voulue dans leur espace intérieur à la manière d'un système alternatif par une mise à vide et un gazage, le couvercle amovible (3) du récipient interchangeable (2) contenant des canaux d'entrée et de sortie de gaz (49, 50) avec chacun un clapet de retenue (51, 52), ou analogue, pour l'échange gazeux.

2. Appareil selon la revendication 1, **caractérisé en ce qu'**il est prévu, comme moyen pour la liaison et la séparation automatisées du dispositif (4) respectivement avec et vis-à-vis de l'espace intérieur du récipient interchangeable (2), un cylindre d'accostage (5) fonctionnant sous vide, qui est formé par une unité piston-cylindre (11, 12), présente un canal interne d'amenée de gaz (19) avec un raccord de tuyauterie de gaz (17) pour le dispositif (4) de production de l'atmosphère voulue et peut être pressé, de façon étanche au gaz, contre une zone du couvercle du récipient interchangeable (3) présentant des entrées et sorties de gaz (49, 50).

3. Appareil selon la revendication 2, **caractérisé en ce que** le cylindre (11) est monté de manière fixe dans l'appareil et que le piston (12) présente du côté récipient un adaptateur d'accostage (13), sortant du cylindre (11), qui peut être déplacé avec le piston (12) contre un ressort de compression coaxial (14) et est muni du côté frontal d'une lèvre d'étanchéité (15) pouvant être pressée contre le couvercle du récipient interchangeable (3).

4. Appareil selon la revendication 3, **caractérisé en ce que** l'adaptateur d'accostage (13) est guidé de manière étanche au gaz dans une paroi frontale (16) côté récipient du cylindre (11) et le ressort de compression (14) est placé dans le cylindre (11) entre le piston (12) et la paroi frontale (16) du cylindre (11).

5. Appareil selon la revendication 3 ou 4, **caractérisé en ce que** le piston (12) est muni, de son côté opposé au récipient interchangeable (2), d'un tourillon de piston (18) présentant le raccord de tuyauterie de gaz (17) du cylindre d'accostage (5).

6. Appareil selon la revendication 5, **caractérisé en ce que** le piston (12), l'adaptateur d'accostage (13) et le tourillon de piston (18) sont disposés de manière coaxiale les uns aux autres et sont traversés par un canal d'entrée et de sortie de gaz (19) orienté dans le sens axial, qui débouche dans le raccord de tuyauterie de gaz (17) du cylindre d'accostage (5).

7. Appareil selon l'une ou plusieurs des revendications 1 à 6, **caractérisé en ce qu'**une première unité de construction (8) avec une pompe à vide (24), une deuxième unité de construction (9) avec au moins une source d'alimentation en gaz (26a, 26b), le cylindre d'accostage (5), des soupapes de commande (29a à 29e) et des régulateurs (31), ainsi qu'une troisième unité de construction (10) avec des boutons-poussoirs (32), signalisations (33) et imprimante (34) sont montées dans l'appareil (1).

8. Récipient interchangeable pour un appareil selon l'une ou plusieurs des revendications 1 à 7, **caractérisé en ce que** le couvercle amovible du récipient interchangeable (3) contient des canaux d'entrée et de sortie de gaz (49, 50) avec chacun un clapet de retenue (51, 52), ou analogue, pour l'échange gazeux via le cylindre d'accostage (5).

9. Récipient interchangeable selon la revendication 8, **caractérisé en ce que** le récipient interchangeable (2) a la forme d'un pot et est muni de nervures verticales (43) disposées sur la périphérie.

10. Récipient interchangeable selon la revendication 9, **caractérisé en ce que** le couvercle du récipient interchangeable est formé par un plateau (44) ajusté de manière étanche au gaz à la circonférence intérieure du récipient interchangeable (45), comportant une bague de fixation (46) entourant le bord du récipient interchangeable (45), une fermeture à baïonnette (47) ou analogue.
